# FASCICULE DE BREVET EUROPEEN

(11) **EP 2 665 703 B1**
(45) Date de publication et mention de la délivrance du brevet: **06.12.2017**
(21) Numéro de dépôt: 12708717.9
(22) Date de dépôt: 20.01.2012
(51) Int. Cl.: C07C 315/02, C07C 317/44

(54) **ÉNANTIOMÈRE (R) DE LA LAUFLUMIDE À PLUS DE 95%ee**
ENANTIOMER (R) VON LAUFLUMID IN MORE THAN 95%ee
ENANTIOMER (R) OF LAUFLUMIDE IN MEHR ALS 95%ee

(30) Priorité: 20.01.2011 FR 1150455
(43) Date de publication de la demande: 27.11.2013
(73) Titulaire: NLS Pharma AG, 6370 Stans (CH)
(72) Inventeur: KONOFAL, Eric, F-60300 Senlis (FR)
(74) Mandataire: Regimbeau
(86) Numéro de dépôt international: PCT/EP2012/050881
(87) Numéro de publication internationale: WO 2012/098235

(56) Documents cités:
- EP-A1- 0 097 071
- WO-A1-2005/028428
- J. CAO ET AL.: "SARs at he monoamine transporters for a novel series of modafinil analogues", ACS MED. CHEM. LETT., vol. 2, 18 octobre 2010 (2010-10-18), pages 48-52, XP002645183,

## Description

L'invention concerne le domaine des médicaments utiles dans le traitement des troubles associés à un déficit de l'attention, de la régulation de l'attention et/ou de l'éveil, en particulier du Trouble Déficit de l'Attention Hyperactivité (TDAH).

Le Trouble Déficit de l'Attention Hyperactivité (TDAH) qui associe une inattention, une impulsivité, et une hyperactivité comportementale est un motif fréquent de consultation en psychopathologie de l'enfant. Sa prévalence dans la population générale des enfants est selon les études de 2 à 5%. Le diagnostic repose sur des critères cliniques définis dans le Manuel Diagnostique et Statistique des Troubles Mentaux (DSM-IV). Le TDAH est avant tout l'expression exagérée, permanente et continue de manifestations comportementales qui ne sont pas dues à une carence éducative, pédagogique ou socio-économique. Les signes d'inattention peuvent persister au-delà de l'enfance et sont responsables des difficultés sociales, relationnelles et affectives.

L'hypothèse selon laquelle l'hyperactivité pourrait être secondaire à un trouble du maintien de l'éveil ou un trouble primaire de la vigilance a été, au cours des vingt dernières années passées bien documentée, et enrichie par plusieurs études montrant l'implication du maintien de l'attention dans l'origine du trouble. Notamment Palm (1992), Lecendreux, Konofal et al. (2000) et Golan et al. (2004) ont pu montrer que les enfants présentant un TDAH avaient une somnolence diurne parfois sévère (valeur moyenne aux tests itératifs d'endormissement courte), et montré qu'ils s'endormaient dans certains cas plus rapidement ou plus fréquemment que les enfants du groupe contrôle lorsque les conditions propices au sommeil étaient proposées (enfant allongé au calme, conditions de bruit et de lumière optimales). Les résultats de ces études menées sur la recherche de dysfonctionnement des mécanismes de l'éveil dans le TDAH de l'enfant, confortant l'hypothèse selon laquelle la somnolence diurne pourrait être sous-jacente des symptômes comportementaux, ont mis en relief certains aspects cliniques de cette pathologie confortant ainsi l'argumentaire sur l'intérêt d'utiliser des molécules eugrégoriques, éveillantes dans le traitement symptomatologique du TDAH.

Les substances stimulantes utilisées dans le traitement pharmacologique du TDAH, en particulier chez les enfants, appartiennent à plusieurs classes pharmacologiques: psychostimulants (amphétamine, méthylphénidate, bupropion), eugrégoriques (Modafinil, Adrafinil), et inhibiteurs de la mono-amine oxydase B (selegiline).

Les plus utilisées et les mieux connues sont:
- le méthylphénidate (MPH) est le traitement de référence du TDAH chez les enfants, les adolescents et les adultes. C'est avant tout un psychostimulant connu pour ses propriétés stimulantes. A part son action dopaminergique stimulante, sur le relargage de la noradrénaline et de la dopamine, par inhibition et recapture, le MPH n'a pas d'effet sur les récepteurs alpha-1 postsynaptiques noradrénergiques (modification de la sensibilité).
- l'amphétamine (D,L-amphetamine) présente une action sur le relargage extra-vésiculaire de la noradrénaline et de la dopamine et par conséquent inhibe toute forme de stockage. A cause de son potentiel usage impropre et de ses effets périphériques indésirables son utilisation reste très limitée voire interdite dans la majorité des pays européens.
- le Modafinil ((±)-2-(benzhydrylsulfinyl)acétamide) est un médicament (Modiodal®) analeptique approuvé par la FDA (Food and Drug administration) pour le traitement de la narcolepsie, de la somnolence diurne excessive résiduelle associée au syndrome d'apnées obstructives du sommeil.

L'EMA (ex-EMEA) ne l'a recommandé que pour la narcolepsie.

Le Modafinil a été montré efficace dans le traitement du TDAH (WO01/12170) mais rejeté par la FDA pour un cas de syndrome de Stevens-Johnson.

Le Modafinil est le métabolite primaire de l'Adrafinil (2-((diphénylméthyl)sulfinyl)-N-hydroxyacétamide) (Olmifon®, Cephalon) qui requiert une dose plus élevée pour aboutir aux mêmes effets. C'est un stimulant (eugrégorique) dont le complexe mécanisme d'action est mal connu. Il ne cause pas de dépendance.
- l'Atomoxetine, un inhibiteur sélectif de la recapture de la noradrénaline, et un stimulant dopaminergique (par inhibition de la recapture au cortex pré-frontal), a démontré l'efficacité et une bonne tolérance dans le TDAH chez les enfants et les adultes (Spencer et al, 1998; Popper 2000; Biederman et al, 2002). Elle a été autorisée par la FDA en Novembre, 2002.
- autres: bupropion, caféine, sélégiline, etc.

Le Bupropion, un inhibiteur de la recapture des catécholamines et un antidépresseur, est également un compétiteur potentiel dans le traitement du TDAH.

La sélégiline, un inhibiteur de la recapture des mono-oxydases, présente également des propriétés pharmacologiques proches de celles des amphétamines. Son action stimulante dans le traitement du TDAH est connue et son avantage dans cette utilisation est possible.

Ainsi, l'amélioration de l'hyperactivité motrice par des psychostimulants dopaminergiques est souvent très significative, mais néanmoins insuffisante.

C'est parce que les substances stimulantes utilisées ou qui pourraient être utilisées dans le traitement du TDAH, en particulier les psychostimulants tels que le méthylphénidate ou les amphétamines, présentent souvent une demi-vie plasmatique courte, qui implique l'apparition d'effets "on-off', c'est-à-dire un effet off accompagné d'un effet rebond après quelques heures et est responsable d'une aggravation des symptômes dans la dernière partie de la nuit, et nuisible à la qualité d'endormissement.

En outre, certaines de ces substances sont métabolisées dans l'organisme et par conséquent présentent un risque toxique pour le patient.

En outre, certains symptômes particuliers tels que l'insomnie, des difficultés à s'endormir, se lever pendant la nuit, éventuellement dus à une agitation motrice nocturne excessive, ainsi que des troubles supplémentaires tels que l'inattention, l'impatience et l'impulsivité, semblent résister à toute forme de traitement [Chervin et al, Sleep 2002 15;25(2):213-8; Gruber et al, J Am Acad Child Adolesc Psychiatry. 2000;39(4):495-501].

La narcolepsie ou *maladie de Gélineau* est une affection neurologique grave caractérisée par deux symptômes principaux que sont la narcolepsie et la cataplexie. Le principal symptôme est la présence quotidienne d'épisodes irrépressibles de sommeil. La cataplexie se manifeste dans 3 cas sur 4 environ. La structure du sommeil est toujours perturbée. Quand une personne souffre de narcolepsie, elle passe directement de l'état d'éveil au sommeil paradoxal sans étape intermédiaire.

D'autres symptômes secondaires peuvent se manifester, comme des hallucinations hypnagogiques ou hypnopompiques, des paralysies du sommeil, des actes automatiques. Elle fait partie des dyssomnies, ou troubles du sommeil.

La narcolepsie peut être diagnostiquée par un EEG.

La prévalence de la narcolepsie est estimée à entre 2 ou 3 pour 10 000 habitants en France.

La cataplexie, qui est une perte brusque du tonus musculaire sans altération de la conscience, n'est pas un symptôme présent chez tous les narcoleptiques. La prévalence de la cataplexie se situe entre 75 et 80 % des narcoleptiques.

La narcolepsie touche un peu plus d'hommes que de femmes. Il existe deux pics principaux d'apparition de la pathologie, soit à l'adolescence, soit vers la quarantaine.

La caractéristique principale de la narcolepsie est une hypersomnolence diurne, due à des accès irrépressibles de sommeil survenant plusieurs fois par jour et durant de quelques secondes à 30 minutes, parfois plus, et parfois dans des situations délicates (file d'attente dans un magasin, voyage en train ou dans un transport en commun, activité au bureau...) voire dangereuses (traversée de la rue, conduite d'une auto ou d'une moto, utilisation d'un outil de bricolage ou professionnel, bain d'un bébé...).

Ces fluctuations continuelles et importantes de la vigilance s'accompagnent de difficultés d'attention et parfois de troubles momentanés de la mémoire. Elles peuvent entraîner des automatismes (phrases sans sens, rangements d'objets dans des endroits insolites, perte continuelles de clés ou de sacs à main, conduite d'une voiture dans un endroit imprévu, rêves éveillés inopinés... et parfois compromettants, etc.). Généralement l'accès de sommeil est réparateur pendant une heure ou deux, puis survient un nouvel accès de sommeil. La journée du narcoleptique est ponctuée ainsi d'alternances d'états de veille et d'accès de sommeil.

Les « hypersomnolences » et les accès de cataplexie peuvent être très handicapants, peuvent réduire voire empêcher toute activité professionnelle ainsi que de nombreuses autres activités. Les malades sont parfois amenés à un isolement social de plus en plus important. Il n'est pas rare que la narcolepsie soit une cause de dépression nerveuse subséquente.
Les traitements existants à ce jour visent seulement l'apaisement des symptômes et non la cause.

Contre le symptôme de l'hypersomnolence les traitements actuels sont des molécules éveillantes comme le modafinil ou stimulantes comme le méthylphénidate, les sels d'amphétamine, ou encore la méthamphétamine.

Contre le symptôme de la cataplexie, l'usage de l'oxybate de sodium (gamma hydroxy-butyrate sodique), qui permet une certaine forme de restauration du sommeil lent profond lorsqu'il est pris au coucher et pendant la nuit, est aujourd'hui le traitement de référence. (Robinson DM, Keating GM. CNS Drugs. 2007;21(4):337-54. Review. Erratum in: CNS Drugs. 2007;21(8):692.)

D'autres molécules peuvent aussi être utilisées. Il s'agit des molécules ayant un rôle inhibiteur de recapture de la noradrénaline et/ou de la sérotonine et/ou de la dopamine. Donc des médicaments connus par ailleurs pour leur rôle antidépresseur (clomipramine, imipramine, ou protriptyline, venlafaxine).

L'hypersomnie idiopathique est une maladie très rare caractérisée par une somnolence diurne excessive dont l'origine est inconnue.

Les manifestations débutent généralement chez l'adulte jeune, avant 30 ans. La fréquence de la maladie, bien que difficile à estimer du fait de sa rareté, toucherait de 1 personne sur 10 000 à 500 000 habitants (5 à 10 fois plus rare que la narcolepsie).

Aucune cause n'est retrouvée à cette hypersomnie, c'est pour cela qu'on la qualifie d'idiopathique.

Il s'agit d'une somnolence diurne excessive quasi-permanente, peu fluctuante au cours de la journée. Cette somnolence oblige le sujet à faire une ou plusieurs siestes dans la journée, qui peuvent alors durer plusieurs heures. Un des éléments clefs du diagnostic clinique, c'est que ces siestes n'ont pas le caractère rafraîchissant qu'elles ont normalement ou dans la narcolepsie (le sujet est toujours aussi fatigué au sortir de cette sieste).

Leur sommeil de nuit est le plus souvent de très bonne qualité, avec très peu d'éveils. Par contre, le réveil est particulièrement difficile (ivresse de sommeil) et on note une inertie au réveil: c'est-à-dire que le sujet est très ralenti sur le plan psychomoteur, pouvant aller jusqu'à un état de confusion.

On note chez les sujets atteints, une fatigue permanente, une diminution des performances mentales, des troubles de la mémoire et des difficultés de concentration.

Ce qui fait la différence avec la narcolepsie, c'est qu'on ne retrouve pas de cataplexies, et rarement des paralysies du sommeil ou des hallucinations hypnagogiques. Officiellement la différence de diagnostique entre hypersomnie et narcolepsie repose sur la présence de cataplexie ou de plusieurs endormissements en sommeil paradoxal lors du TILE.
Les TILE (Test itératif de latence à l'endormissement) montrent un endormissement pendant la journée, celui-ci n'est pas toujours anormalement rapide, mais il se fait toujours en l'absence de sommeil paradoxal (SOREM).

Le traitement permet en général aux patients de conduire une activité professionnelle normale en levant les gênes liées au réveil aux accès de sommeil diurnes. Il repose comme pour la narcolepsie sur le modafinil, ou les dérivés amphétaminiques comme le méthylphénidate mais en cas d'échec on peut essayer d'autres traitements comme le Teronac® (mazindol) ou la Dexamine® (dextro-amphétamine). Des antidépresseurs stimulants peuvent être utiles (Prozac®, Effexor®). Les conseils d'hygiène de sommeil, notamment l'interdiction des siestes font également partie des outils pouvant soulager le handicap créé par cette maladie mais ceux-ci restent parfois inefficaces.

Il existe donc un réel besoin de développer de nouveaux traitements du TDAH, de la narcolepsie et de l'hypersomnie idiopathique qui donneraient de meilleurs résultats que ceux obtenus avec les traitements actuels à base de psychostimulants et traiteraient les symptômes résistants aux traitements actuels, sans effet rebond des symptômes et présenteraient une faible toxicité.

La demande EP 0 097 071 décrit le mélange racémique de la Lauflumide (composé CRL 40940) comme étant un agent stimulant (p. 2, 1. 20). Ce composé a également donné d'excellents résultats en tant que médicament de l'éveil et agit efficacement contre l'énurésie (p. 26,1. 30-36). L'article de Cao et al. (Med. Chem. Lett., 2, 48-52, 2011) décrit une nouvelle série d'analogues du Modafinil présentant une activité psychostimulante (p. 51, col. 2, § 2). Le composé 5b de cette série représente le mélange racémique de la Lauflumide. La demande internationale WO 2005/028428 décrit un procédé de synthèse énantiosélectif du Modafinil avec une étape finale d'oxydation asymétrique. L'exemple 16 (Entrée 2, Tableau 17, p. 41) correspond au précurseur prochiral de la Lauflumide sans divulguer la configuration de l'énantiomère obtenu. Toutefois, aucun de ces documents ne décrit explicitement l'utilisation de la Lauflumide dans le traitement du TDAH, ni l'énantiomère (R) de la lauflumide.

De façon surprenante et inattendue, les inventeurs ont synthétisé une molécule proche du Modafinil et de l'Adrafinil et montré qu'elle est plus efficace que ces deux molécules dans leurs indications avec moins d'effets secondaires.

Cette molécule est la (R)-Lauflumide ((R)-2-((bis(4-fluorophenyl)methane)sulfinyl)acétamide

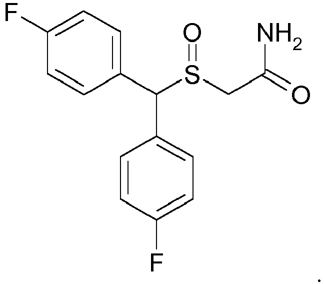

Cette molécule présente un centre chiral au niveau du groupement sulfoxyde -S=O.

Par conséquent, l'invention concerne tout d'abord la molécule de Lauflumide, dans sa forme d'énantiomère (R), optiquement pur en excès énantiomérique à plus de 95%.

L'énantiomère dextrogyre correspond à l'énantiomère R.

Selon la présente invention et lorsqu'il n'est pas précisé autrement, on entend par « Lauflumide » la Lauflumide dans sa forme d'énantiomère (R), optiquement pur en excès énantiomérique à plus de 95%.

Avantageusement, l'invention concerne l'un des énantiomères de la lauflumide optiquement pur en excès énantiomérique à plus de 96%, de manière encore préférée à plus de 97%, de manière plus préférée à plus de 98%, de manière particulièrement préférée à plus de 99%. Cet énantiomère est la D-lauflumide ou (+)-Lauflumide ou Dextro-Lauflumide (ou encore dexlauflumide).

La (R)-Lauflumide est plus efficace que le mélange racémique de Lauflumide.

La Lauflumide n'est pas une amphétamine. Elle ne présente par conséquent pas les effets secondaires des amphétamines.

Son efficacité est de 20 fois supérieure à celle de l'adrafinil et 4 fois supérieure à celle du modafinil.

La Lauflumide constitue une alternative thérapeutique au méthylphénidate et à l'amphétamine dans le TDAH et au modafinil dans la narcolepsie et l'hypersomnie idiopathique.

La durée d'efficacité plasmatique attendue de la Lauflumide (mélange racémique) est de 6 à 7 heures. Seules les associations des méthylphénidates (libération immédiate et libération prolongée) peuvent parvenir à traiter le TDAH sur la journée. Il en est de même avec l'amphétamine et le modafinil. Cependant, ces associations nécessitent une parfaite logistique (horaires de prises et de dispensations), et constituent une réelle limite d'efficacité dans le temps. La Lauflumide est donc une authentique alternative aux molécules jusqu'à maintenant labélisées dans le TDAH, la narcolepsie et l'hypersomnie idiopathique.

La présente invention consitute donc une réelle innovation tant sur le plan thérapeutique, que sur le plan psycho-social.

Un autre objet de l'invention concerne la (R)-Lauflumide ((R)-2-((bis(4-fluorophenyl)methane)sulfinyl)acétamide sous forme d'énantiomère (R), optiquement pur en excès énantiomérique à plus de 95%, en tant que médicament.

Le patient selon l'invention est choisi dans le groupe constitué des nourrissons, des enfants, des adolescents et des adultes.

Selon un aspect de l'invention, la Lauflumide est utilisée en tant que médicament dans un régime de dose de 1 à 600 mg par jour, de préférence dans un régime de dose de 50 à 400 mg par jour.

Un autre objet de l'invention concerne la (R)-Lauflumide ((R)-2-((bis(4-fluorophenyl)methane)sulfinyl)acétamide sous forme d'énantiomère (R), optiquement pur en excès énantiomérique à plus de 95%,pour son utilisation dans le traitement du TDAH.

Un autre objet de l'invention concerne l'utilisation de la (R)-Lauflumide ((R)-2-((bis(4-fluorophenyl)methane)sulfinyl)acétamide sous forme d'énantiomère (R), optiquement pur en excès énantiomérique à plus de 95%,pour la fabrication d'un médicament destiné au traitement du TDAH.

Dans le contexte de l'invention, le diagnostic du TDAH est basé sur les caractéristiques cliniques définies par la classification internationale, DSM/IV (Diagnostic and Statistical Manual of Mental Disorders, 4th ed, 1994).

Les critères du DSM-IV comprennent trois dimensions (inattention, impulsivité et hyperactivité), une efficacité intellectuelle normale (QI>80, pour un âge entre 5 et 12 ans), une carence en fer mais pas d'anémie donc un taux d'hémoglobine normal.

"Symptôme du TDAH" signifie les symptômes premiers du DSM-IV à savoir l'inattention, l'impulsivité et l'hyperactivité motrice diurne et nocturne, et les symptômes secondaires comme l'impatience, les troubles oppositionnels, le syndrome des jambes sans repos, et l'insomnie.

Insomnie signifie:
a. des difficultés à s'endormir;
b. une hyperactivité motrice nocturne et des réveils pendant la nuit, et
c. une insomnie psychopathologique, généralement chronique et liée à l'anxiété, au stress et des épisodes dépressifs.

Selon un aspect de l'invention, la Lauflumide est utilisée dans le traitement du TDAH en combinaison avec du fer en tant que produit de combinaison en administration simultanée, séparée ou séquentielle.

Préférentiellement, le fer est utilisé avant l'administration de la Lauflumide.

Selon la présente invention, "fer" signifie fer sous la forme d'atome de fer, de sel de fer ou de fer organique, ou toute formulation contenant du fer pharmaceutiquement acceptable. Parmi les sels de fer pharmaceutiquement acceptables on peut citer les sels ferreux et les sels ferriques, de préférence le citrate d'ammonium ferrique, pyrophosphate ferrique, ferrocholinate, ascorbate ferreux, aspartate ferreux, chlorure ferreux, sulfate ferreux, tartrate ferreux, fumarate ferreux, ferreux gluconate gluceptate, ferreux, sulfate de glycine ferreux, lactate ferreux, oxalate ferreux et succinate ferreux.

De façon alternative, le fer pharmaceutiquement acceptable est sous la forme de fer dextran, de fer sucrose, de fer poly-maltose, ou de fer sorbitol.

Selon un aspect de l'invention, la Lauflumide est utilisée dans le traitement du TDAH en combinaison avec au moins un composé choisi parmi les psychostimulants en tant que produit de combinaison en administration simultanée, séparée ou séquentielle.

Des psychostimulant appropriés sont alors la dopamine et/ou des inhibiteurs de la recapture de la noradrénaline et des agonistes des catécholamines. Parmi ceux-ci, on peut citer de façon non-exhaustive:
1) psychostimulants: méthylphénidate, modafinil, atomoxétine, et amphétamines telles que la d-amphétamine, la dexadrine et la dexamphetamine.
2) L-Dopa: lévodopa
3) agonistes sélectifs de la dopamine: pramipexole, ropinirole, lisuride, pergolide, cabergoline, etc.

Quand la Lauflumide est utilisée en association avec du fer, le régime de doses du fer est de 50 à 200 mg de fer élément par jour pendant 3 mois (si la ferritine est <30 µg/L).

Quand la Lauflumide est utilisée en association avec un psychostimulant, le régime de doses du psychostimulant est de 10-30 mg/j de méthylphénidate, de 100 à 400 mg/j de modafinil. L'association avec un sel de l'amphétamine est déconseillée.

La présente description décrit également la Lauflumide pour son utilisation médicale comprenant l'administration d'une quantité efficace de Lauflumide à un patient souffrant du TDAH, de préférence à raison de 1 à 400 mg par jour, de manière encore préférée dans un régime de dose de 200 à 300 mg par jour.

La présente description décrit également la Lauflumide pour son utilisation dans le traitement de la narcolepsie ou de l'hypersomnie idiopathique.

La présente description décrit également l'utilisation de la Lauflumide pour la fabrication d'un médicament destiné au traitement de la narcolepsie ou de l'hypersomnie idiopathique.

La présente description décrit également la Luflumide pour son utilisation médicale comprenant l'administration d'une quantité efficace de Lauflumide à un patient souffrant de la narcolepsie ou de l'hypersomnie idiopathique, de préférence à raison de 1 à 400 mg par jour, de manière encore préférée dans un régime de dose de 200 à 300 mg par jour.

Selon une variante de cette méthode, la Lauflumide est utilisée dans le traitement de la narcolepsie ou de l'hypersomnie idiopathique en combinaison avec au moins un composé choisi parmi les antidépresseurs sérotoninergiques et le gamma hydroxyburyrate sodique en tant que produit de combinaison en administration simultanée, séparée ou séquentielle.

Cette administration combinée sera réalisée dans les même conditions d'administration, et dans les mêmes schémas posologiques que ceux proposés avec le modafinil. (Billiard M. et al.; EFNS Task Force. Eur J Neurol. 2006;13(10):1035-48 et Greenhill LL. Et al.; American Academy of Child and Adolescent Psychiatry. J Am Acad Child Adolesc Psychiatry. 2002;41(2 Suppl):26S-49S. Review.)

Un autre objet de l'invention concerne une composition pharmaceutique comprenant la Lauflumide en tant que principe actif et un ou plusieurs excipients pharmaceutiquement acceptables.

Les composés ou compositions selon l'invention peuvent être administrés de différentes façons et sous différentes formes bien connues de l'homme du métier. Ainsi, ils peuvent être administrés de façon systémique, orale, anale ou parentérale, par exemple par voie intraveineuse, intramusculaire, sous-cutanée, transdermique ou intra-artérielle. De préférence, ils sont administrés par voie orale.

Pour les injections, les composés se présentent généralement sous forme de suspensions liquides. Ils peuvent également être administrés sous forme de gels, huiles, comprimés, suppositoires, poudres, gélules, aérosols, etc, éventuellement au moyen de formes ou dispositifs galéniques à libération prolongée.

Les compositions pharmaceutiques de l'invention peuvent également comprendre du fer ou un ou plusieurs psychostimulants en tant que principe actif.

Un autre objet de l'invention concerne un produit pharmaceutique comprenant la Lauflumide et du fer ou un ou plusieurs psychostimulants en tant que principes actifs en tant que produit de combinaison pour une administration simultanée, séparée ou séquentielle.

Un autre objet de l'invention concerne un procédé de préparation de la Lauflumide comprenant les étapes successives suivantes :
(i) préparation du réactif de Grignard 2
(ii) préparation du fluorobenzhydrol 4 par condensation du réactif de Grignard 2 avec le fluorobenzaldehyde 3 ou le formiate d'éthyle 10
(iii) préparation de l'imidate 5 par ajout de thiourée au fluorobenzhydrol 4
(iv) saponification de l'imidate 5 et formation du thiolate de sodium 6
(v) ajout du chloroacétamide 7 et isolation du composé 8
(vi) oxydation du soufre du composé 8 en sulfoxyde.

Préférentiellement, l'étape (ii) se déroule à -18°C.

Préférentiellement, l'étape (iii) est réalisée par ajout de thiourée avec HBr.

Préférentiellement, l'étape (iii) est réalisée à 60°C et le précipité 5 est obtenu par chauffage à 80°C puis refroidissement à température ambiante.

Préférentiellement, le précipité 5 est filtré et lavé entre l'étape (iii) et l'étape (iv).

Préférentiellement, l'imidate 5 est saponifié à l'étape (iv) en solution aqueuse par ajout de soude concentrée.

Préférentiellement, l'oxydation à l'étape (vi) du soufre en sulfoxyde est réalisée avec de l'eau oxygénée dans l'acide acétique.

Eventuellement, l'étape (vi) est suivie d'une étape (vii) de précipitation de la lauflumide 9 après dilution à l'eau du milieu réactionnel obtenu après l'étape (vi).

Eventuellement, l'étape (vii) est suivie d'une étape (viii) de cristallisation de la lauflumide 9, préférentiellement dans un mélange MeOH/H₂O.

Les figures et exemples suivants illustrent l'invention sans en limiter la portée.
Figure 1 : Schéma du test du labyrinthe en T. c0, c1 et c2 = limites des portes guillotine.
Figure 2 : Graphique de représentation des résultats des tests du labyrinthe en T (Exemple 2)
Légende : Avant = session avant le médicament
Sous = session sous médicament
Après = session après le médicament

### Exemple 1 : Synthèse de la lauflumide

La synthèse commence par la préparation du réactif de Grignard 2 à partir du bromure aromatique 1 avec du magnésium dans l'éther. A la solution du Grignard 2 est ajouté à -18°C le fluorobenzaldehyde 3 pour fournir le fluorobenzhydrol 4. Il est à noter qu'au lieu d'utiliser le fluorobenzaldehyde 3, il serait possible d'utiliser le formiate d'éthyle pour condenser 2 unités de Grignard 2. Cette option est intéressante économiquement le fluorobenzaldehyde 3 étant relativement onéreux.

A une solution aqueuse préformée de thiourée avec HBr est additionné le fluorobenzhydrol 4 à 60 °C. Après chauffage jusqu'à 80°C la solution est laissée refroidir à température ambiante puis le précipité 5 est filtré et lavé avec de l'eau froide. Le précipité 5 est ensuite suspendu dans une solution aqueuse puis de la soude concentrée fraichement préparée est ajoutée au mélange pour saponifier l'imidate 5. Le thiolate de Sodium 6 est formé et après addition du chloroacétamide 7 le composé 8 peut être isolé.

L'oxydation du soufre en sulfoxyde est réalisée avec de l'eau oxygénée dans l'acide acétique. Après réaction, le milieu réactionnel est dilué à l'eau et la lauflumide 9 précipite assez rapidement. La lauflumide 9 est ensuite recristallisé dans un mélange MeOH/H₂O.

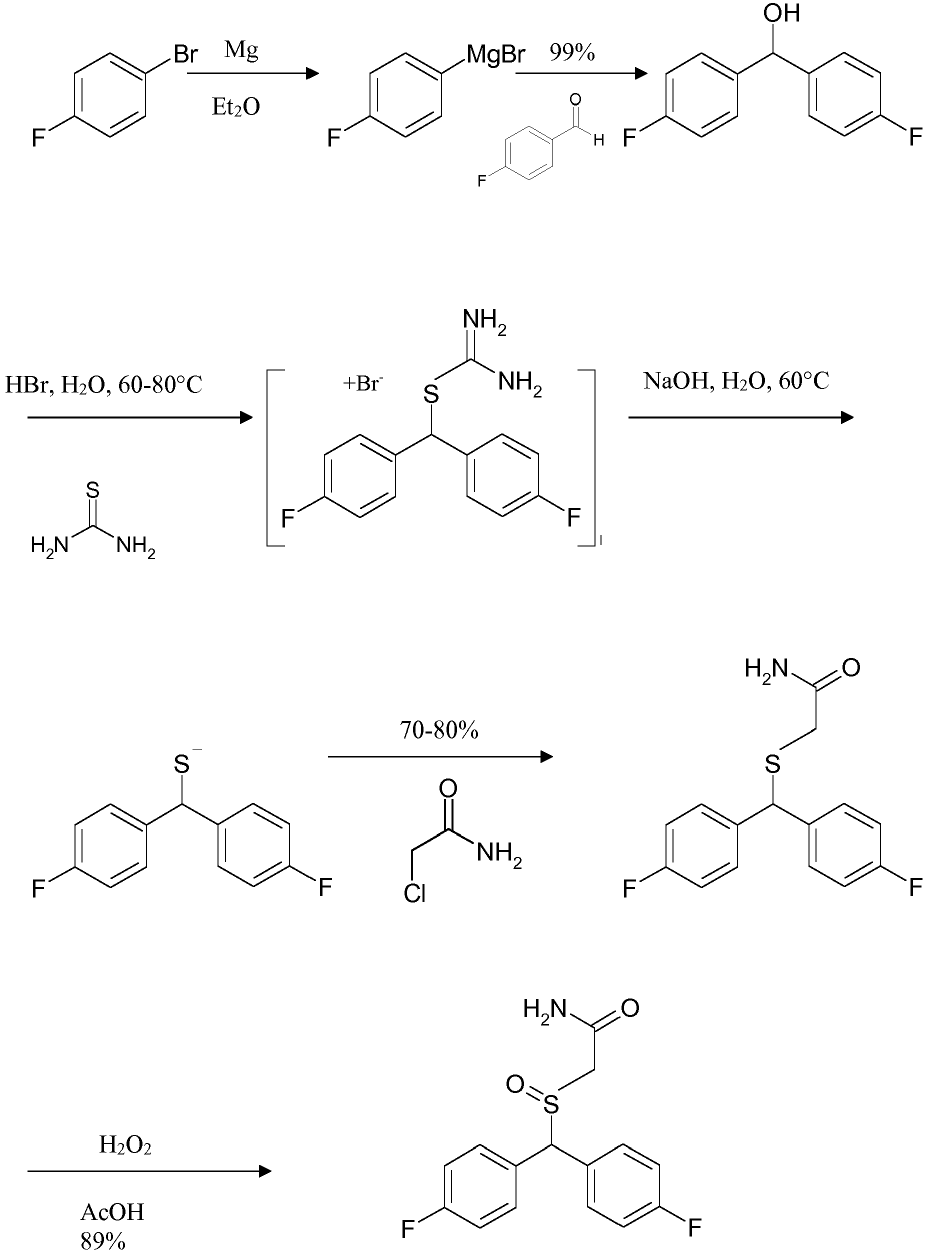

### Exemple 2 : Effets de la lauflumide comparativement avec le Mazindol et le Modafinil dans le modèle TDAH

### Principe

L'impulsivité est un symptôme du TDAH.

Le test de la capacité à attendre dans un labyrinthe en T consiste à soumettre un rat à un choix entre une récompense alimentaire rapide et immédiate et une récompense importante mais différée. Chez les rats Wistar adultes, de nombreux anti-dépresseurs, comme les inhibiteurs de la recapture de la sérotonine ou de la noradrénaline, augmentent le nombre de choix pour la récompense importante mais différée, indiquant une amélioration de la capacité d'attente, c'est-à-dire une diminution de l'impulsivité [2,3].

De précédents résultats ont montré que le méthylphénidate augmentait le nombre de choix pour la récompense importante mais différée chez les jeunes rats Wistar, mais pas chez les adultes Wistar, Spontaneously Hypertensive Rats (SHR) et Wistar Kyoto (WKY) [4]. Ces résultats ont été confirmés par les découvertes qu'à la fois les médicaments stimulants - méthylphénidate et amphétamine , les inhibiteurs de la recapture de la noradrénaline non-stimulants- atomoxetine et desipramine - et un agoniste des récepteurs alpha-2 adrénergiques - la clonidine - amélioraient la capacité à attendre des jeunes rats soumis au test du labyrinthe T [5,6]. Etant donné que ces médicaments réduisent les symptômes y compris l'impulsivité du TDAH [7], ces données indiquent que le test du labyrinthe en T chez les jeunes animaux est approprié pour tester l'amélioration du contrôle de l'impulsivité par des médicaments pour le traitement du TDAH.

Le but de la présente étude est d'examiner si la Lauflumide sous sa forme de mélange racémique ou sous sa forme D (énantiomère (+)), administré à 3 doses avant le test, améliore la capacité d'attente chez les jeunes rats Wistar soumis au test du labyrinthe en T. Une amélioration de la capacité d'attente par ce composé indiquerait que ce composé réduit l'impulsivité et par conséquent pourrait être utile dans le traitement du TDAH. Le méthylphénidate et la d-amphétamine sont utilisés en tant que produit de référence dans la même expérience.

### Matériel et Méthodes

Toutes les expériences sont réalisées entre 8h et 18h à température ambiante (22 ± 1,5°C) sous éclairage artificiel dans des conditions calmes.

### 1. Animaux

On utilise des rats AF Wistar males (Centre d'Elevage René Janvier, France), de 22 jours au début de l'expérience et entre 30 et 42 jours au moment de l'administration des molécules.
Nombre d'animaux : 100 répartis en 3 lots
Température: 22±1.5°C.
Hygrométrie: 50±25%.
Lumière : 50 Lux; 7h-19h
Alimentation: A04 (Safe, France); 5-10 g/jour/animal.
Eau disponible *ad libitum*

### 2. Molécules testées

Mazindol : 1, 3 et 10 mg/kg par voie intrapéritonéale
Méthylphénidate : 3 mg/kg par voie intrapéritonéale
D-amphétamine : 1 mg/kg par voie intrapéritonéale
Modafinil : 64, 128 mg/kg par voie orale
Lauflamide : 64, 128 mg/kg par voie orale
D-Lauflumide : 64, 128, 256 mg/kg par voie orale
Placebo : 1 mL/kg par voie intrapéritonéale

### 3. Matériel

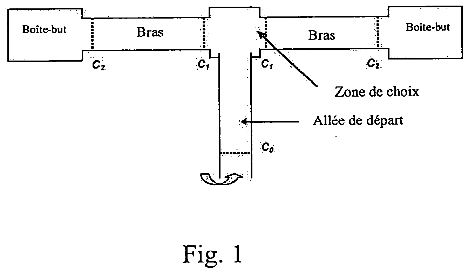

Les expériences ont été réalisées dans deux dispositifs de labyrinthe en T identiques (Fig. 1) en tube de plastique gris opaque (diamètre interne: 7,5 cm), consistant en une allée de départ (30 cm de long), une boîte en plastique transparent (10 cm de large, 10 cm de profondeur, 10 cm de haut) et deux bras (35 cm de long) chacun menant à une boîte rectangulaire en plastique noir (18 cm de large, 30 cm de profondeur, 10 cm de haut). Les portes guillotine amovibles en plastique gris peuvent être insérées dans des fentes verticales, situées à l'entrée de l'allée de départ et à chaque extrémité des bras. Une des boîtes-but (gauche ou droite, suivant les rats) est constamment fournie d'une récompense importante, l'autre d'une petite récompense. Les récompenses importantes et petites consistent respectivement, en 5 et 1 boulettes (20 mg, Technical & Scientific Equipment GmbH, Germany). Les boulettes sont placées dans une coupelle translucide avant chaque essai.

### 4. Protocole expérimental labyrinthe en T

Le test du labyrinthe utilisé est un test validé (T-maze) qui permet, par extrapolation à l'homme, de vérifier l'efficacité des agents pharmacologiques efficaces dans le TDAH

### Phase d'entraînement

*1^{ère} phase- accoutumance.* Les animaux sont d'abord soumis à deux à six sessions de 5 min d'accoutumance. Le rat est délicatement introduit dans l'allée de départ, qui est ensuite fermée avec une porte guillotine insérée dans la fente (références c0 à la Figure 1). On laisse l'animal explorer librement le dispositif et manger des récompenses placées dans des coupelles.

*2^{nde} phase-pré-entraînement.* Après qu'une porte ait été placée dans la fente c2 près de chaque boîte-but, le rat est introduit dans l'allée de départ. Quand il entre dans un des deux bras, une porte est insérée derrière lui dans la fente c1 près de la zone de choix et la porte placée dans la fente c2 est ôtée. Dès que l'animal entre dans la boîte-but, la porte est replacée dans la fente c2. Le rat est récupéré de la boîte-but dès qu'il a mangé les boulettes. L'animal est ensuite remis dans sa cage pour 2 à 3 min. Chaque rat est soumis une à trois fois/jour à cinq sessions d'essais. En 4 à 12 sessions, le rat choisit le bras donnant accès à la récompense importante dans plus de 80% des essais. L'entraînement débute alors.

*3ème phase: entraînement.* Dès lors que les rats suivent 1 à 4 fois/jour des séances d'entraînement de 5 essais pendant lesquels un délai est introduit avant l'accès à la récompense importante. Après qu'une porte ait été placée dans la fente c2 près de chaque boîte-but, le rat est introduit dans l'allée de départ. Quand il entre dans un des deux bras, une seconde porte est insérée derrière lui dans la fente c1 près de la zone de choix, de sorte que le rat choisissant le bras menant à la récompense importante puisse être détenu dans ce bras pendant 30 s -le délai d'attente- avant d'avoir accès au renforcement. Sinon, si l'animal choisit le bras menant à la petite récompense, la porte placée dans la fente c2 est immédiatement ouverte, permettant à l'animal d'entrer dans la boîte-but. Tester les médicaments commence quand l'animal choisit la récompense importante et retardée de 30 s à 2 essais sur 5 (ou moins) pendant deux sessions consécutives et à 1 essai sur 5 (ou moins) à la session suivante. Les animaux qui n'entrent pas dans ce critère pendant 12 sessions sont éliminés de l'expérience.

### Phase de test

Les sessions test sont les suivantes:
- deux sessions avant le médicament,
- deux sessions sous médicament,
- deux sessions de contrôle après le médicament.

Deux sessions de contrôle avant le médicament sont réalisées le même jour, 2 à 4h d'intervalle. Les sessions sous médicament 1 et 2 sont réalisées un et deux jours, respectivement, après les sessions de contrôle avant le médicament. Les deux sessions de contrôle après le médicament sont réalisées un jour après la session sous médicament 2. Le mazindol, le méthylphénidate, la d-amphétamine ou le placebo sont administrés avant chaque session sous médicament.

Les animaux sont répartis au hasard en 10 groupes (n = 10 animaux/groupe) et reçoivent un total de deux administrations IP (une avant chaque session sous médicament) de:
- groupe véhicule : véhicule de mazindol 60 min avant le test
- groupe Maz-1 : mazindol (1 mg/kg) 60 min avant le test,
- groupe Maz-3 : mazindol (3 mg/kg) 60 min avant le test,
- groupe Maz-10 : mazindol (10 mg/kg) 60 min avant le test,
- groupe Mph 3 : méthylphénidate (3 mg/kg) 30 min avant le test,
- groupe Modaf-64 : modafinil (64 mg/kg) 120 min avant le test,
- groupe Modaf-128 : modafinil (128 mg/kg) 120 min avant le test,
- groupe Lauf 64 : lauflumide (64 mg/kg) 120 min avant le test,
- groupe Lauf 128 : lauflumide (128 mg/kg) 120 min avant le test,
- groupe D-A 1 : d-amphétamine (1 mg/kg) 30 min avant le test
- groupe D-Lauf 64 : D-Lauflumide (64 mg/kg) 120 min avant le test
- groupe D-Lauf 128 : D-Lauflumide (128 mg/kg) 120 min avant le test
- groupe D-Lauf 256 : D-Lauflumide (256 mg/kg) 120 min avant le test.

### 5. Analyse des données

Pour chaque animal, le pourcentage de choix de récompense importante mais différée est calculé.

### 6. Résultats

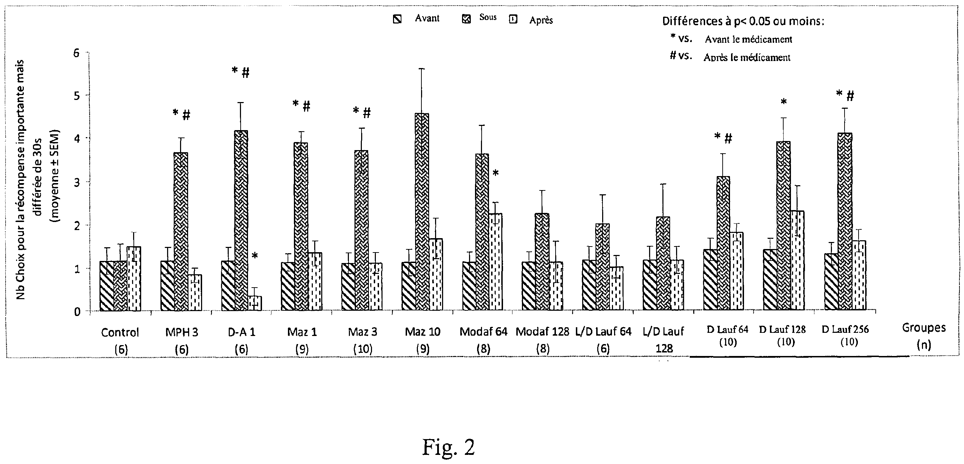

Les résultats sont compilés à la Figure 2.

Le méthylphénidate et la d-amphétamine sont utilisés en tant que produit de référence dans la même expérience.

Le mazindol, la Lauflumide sous sa forme de mélange racémique ou sous sa forme D (énantiomère (+)), administré à 3 doses avant le test, améliorent la capacité d'attente chez les jeunes rats Wistar soumis au test du labyrinthe en T par rapport au contrôle.

Le mazindol et la D-Lauflumide sont au moins aussi efficaces que le méthylphénidate et la d-amphétamine pour améliorer la capacité d'attente chez les jeunes rats Wistar soumis au test du labyrinthe en T.

Le mazindol et la D-Lauflumide sont plus efficaces que le modafinil pour améliorer la capacité d'attente chez les jeunes rats Wistar soumis au test du labyrinthe en T.

### Exemple 3 : Préparation de la D-Lauflumide ((+)-Lauflumide) ou (R)-Lauflumide

A une solution de 2-(bis(4-fluorophenyl)méthylthio)acétamide (9.7 g, 33,1 mmol, 1 equiv) dans du toluène sec (100 mL) à 55 °C est ajouté du (S,S)-(-)-tartrate de diéthyle (1,14 mL, 6,62 mmol, 0,2 equiv) suivi de Ti(O*i*Pr)₄ (890 µL, 3.31 mmol, 0,1 equiv) et d'eau (30µL, 1.66 mmol, 0,05 equiv). Après 50 min d'agitation à 55°C, le milieu réactionnel est refroidi jusqu'à température ambiante, puis de la diisopropyléthylamine (590 µL, 3,31 mmol, 0,1 equiv) est ajoutée suivi d'hydropéroxyde de cumène 80% (6.52 mL, 39.5 mmol, 1.2 equiv). La réaction est agitée 2 à 3h jusqu'à conversion totale du produit de départ en sulfoxyde. La réaction est hydrolysée par une solution à 3% d'acide citrique dans l'acétone (50 mL). Après 20 min d'agitation le milieu réactionnel est filtré, puis concentré sous vide. Le résidu est purifié par colonne chromatographique de silice (AcOEt :Ether de Pétrole 50 :50 puis AcOEt 100%). Une recristallisation dans un mélange méthanol/eau 50 :50 suivi d'un refroidissement à -18°C permet d'obtenir une fraction cristallisée. Le surnageant est concentré et séché sous vide pour fournir le (*R*)-Lauflumide avec un excès énantiomérique de 94% (3.0 g, 29%).

### REFERENCES

Billiard M, Bassetti C, Dauvilliers Y, Dolenc-Groselj L, Lammers GJ, Mayer G, Pollmächer T, Reading P, Sonka K; EFNS Task Force. Eur J Neurol. 2006;13(10):1035-48. *EFNS guidelines on management of narcolepsy.*
Chervin et al, Associations between symptoms of inattention, hyperactivity, restless legs, and periodic leg movements. Sleep 2002 15;25(2):213-8
Golan N, Shahar E, Ravid S, Pillar G. Sleep disorders and daytime sleepiness in children with attention-deficit/hyperactivity disorder. Sleep. 15;27:261-6; 2004
Greenhill LL, Pliszka S, Dulcan MK, Bernet W, Arnold V, Beitchman J, Benson RS, Bukstein O, Kinlan J, McClellan J, Rue D, Shaw JA, Stock S; American Academy of Child and Adolescent Psychiatry. J Am Acad Child Adolesc Psychiatry. 2002;41(2 Suppl):26S-49S. Review. *Practice parameter for the use of stimulant medications in the treatment of children, adolescents, and adults.*
Gruber et al, Instability of sleep patterns in children with attention-deficit/hyperactivity disorder. J Am Acad Child Adolesc Psychiatry. 2000;39(4):495-501
Lecendreux M, Konofal E, Bouvard M, Falissard B, Mouren-Simeoni M-C - Sleep and alertness in children with TDAH. J Child Psychol Psychiatry 41, 6, 803-12;2000
Palm L, Persson E, Bjerre L, Elmqvist D - Sleep and wakefulness in preadolescent children with deficits in attention, motor control and perception. Acta Paediatr 81, 618-24; 1992
Robinson DM, Keating GM. CNS Drugs. 2007;21(4):337-54. Review. Erratum in: CNS Drugs. 2007;21(8):692. *Sodium oxybate: a review of its use in the management of narcolepsy*
Weinberg AS, Brumback RA - Primary disorder of vigilance: a novel explanation of inattentiveness, daydreaming, boredom, restlessness, and sleepiness. J Pediatr 116, 720-5; 1992
Weis M, Murray C, Weiss G. Adults with attention-deficit/hyperactivity disorder: current concepts. J Psychiatr Pract. 28, 99-111; 2002

## Revendications

1. Molécule de Formule (I) suivante : 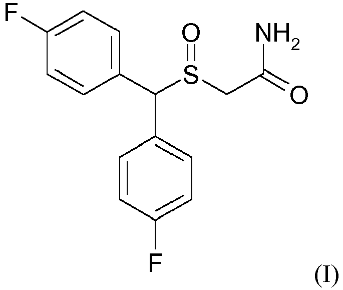 sous sa forme d'énantiomère (R) optiquement pur en excès énantiomérique à plus de 95%.

2. Molécule selon la revendication 1 sous sa forme d'énantiomère (R) optiquement pur en excès énantiomérique à plus de 97%.

3. Molécule selon la revendication 1 sous sa forme d'énantiomère (R) optiquement pur en excès énantiomérique à plus de 99%.

4. Molécule selon l'une quelconque des revendications 1 à 3 pour son utilisation en tant que médicament.

5. Molécule pour son utilisation selon la revendication 4 en tant que médicament dans un régime de dose de 1 à 400 mg par jour.

6. Molécule pour son utilisation selon la revendication 4 ou 5 dans le traitement du Trouble Déficit de l'Attention Hyperactivité et/ou de ses symptômes associés ou comorbides.

7. Molécule pour son utilisation selon la revendication 6 chez l'enfant.

8. Molécule pour son utilisation selon la revendication 4 ou 5 dans le traitement de la narcolepsie ou de l'hypersomnie idiopathique.

9. Molécule pour son utilisation selon l'une quelconque des revendications 6 à 8 en tant que médicament dans un régime de dose de 200 à 300 mg par jour.

10. Composition pharmaceutique comprenant la molécule selon l'une quelconque des revendications 1 à 3 en tant que principe actif et un ou plusieurs excipients pharmaceutiquement acceptables.

11. Composition pharmaceutique selon la revendication 10, adaptée à une administration orale.

12. Composition pharmaceutique selon la revendication 11, sous forme de comprimé.

13. Composition pharmaceutique comprenant la molécule de formule (I) selon l'une quelconque des revendications 1 à 3 et du fer ou un ou plusieurs psychostimulants ou un ou plusieurs antidépresseurs sérotoninergiques ou le gamma hydroxyburyrate sodique en tant que principe actif.

14. Produit pharmaceutique comprenant la molécule de formule (I) selon l'une quelconque des revendications 1 à 3et du fer ou un ou plusieurs psychostimulants ou un ou plusieurs antidépresseurs sérotoninergiques ou le gamma hydroxyburyrate sodique en tant que principes actifs en tant que produit de combinaison pour une administration simultanée, séparée ou séquentielle.

15. Procédé de préparation de la molécule selon l'une quelconque des revendications 1 à 3, comprenant les étapes suivantes :
(i) préparation du réactif de Grignard 2
(ii) préparation du fluorobenzhydrol 4 par condensation du réactif de Grignard 2 avec le fluorobenzaldehyde 3 ou le formiate d'éthyle 10
(iii) préparation de l'imidate 5 par ajout de thiourée au fluorobenzhydrol 4
(iv) saponification de l'imidate 5 et formation du thiolate de sodium 6
(v) ajout du chloroacétamide 7 et isolation du composé 8
(vi) oxydation du soufre du composé 8 en sulfoxyde.

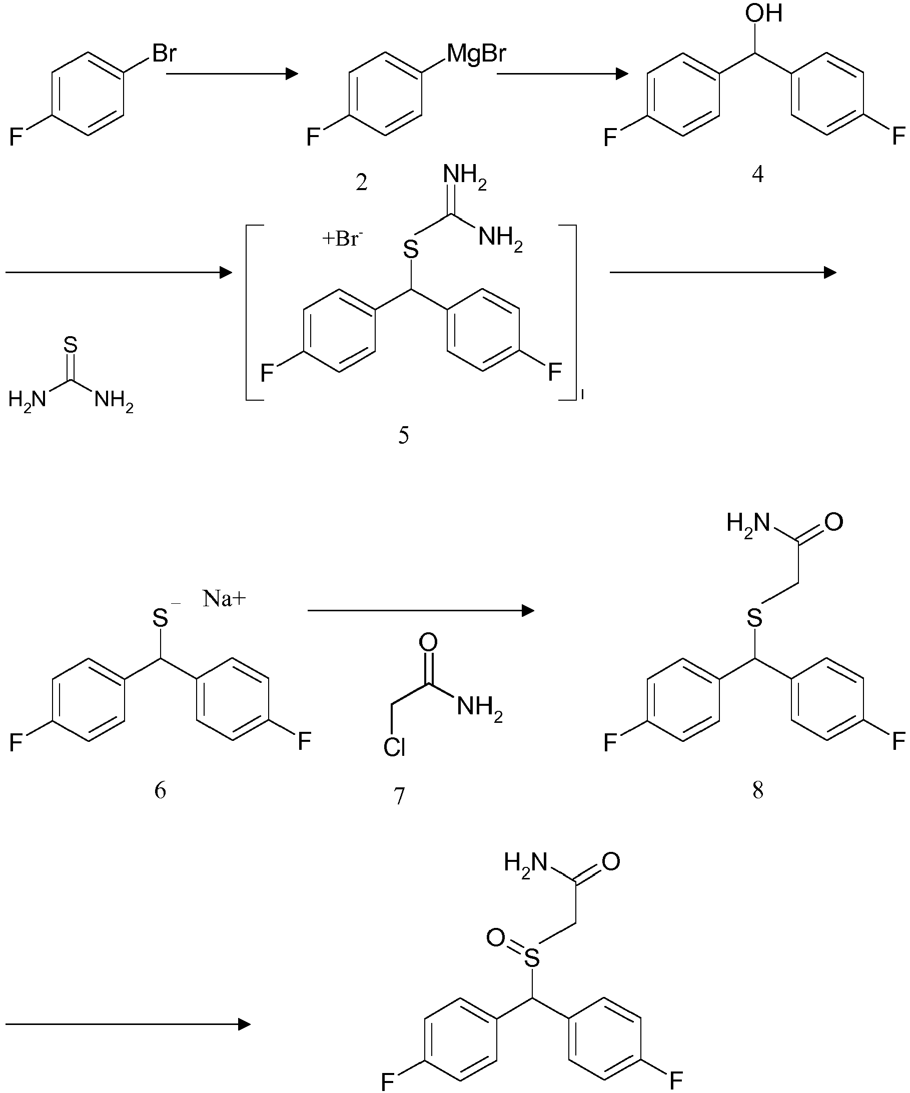

## Patentansprüche

1. Molekül der folgenden Formel (I): 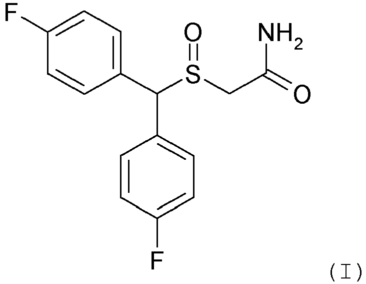 in seiner Form von optisch reinem Enantiomer (R) mit Enatiomerüberschuss von über 95 %.

2. Molekül nach Anspruch 1 in seiner Form von optisch reinem Enantiomer (R) mit Enatiomerüberschuss von über 97 %.

3. Molekül nach Anspruch 1 in seiner Form von optisch reinem Enantiomer (R) mit Enatiomerüberschuss von über 99 %.

4. Molekül nach einem der Ansprüche 1 bis 3 für seine Anwendung als Arzneimittel.

5. Molekül für seine Anwendung nach Anspruch 4 als Arzneimittel in einer Dosierung von 1 bis 400 mg pro Tag.

6. Molekül für seine Anwendung nach Anspruch 4 oder 5 zur Behandlung der Hyperaktivitäts-Aufmerksamkeitsstörung und/oder von ihren verbundenen oder comorbiden Symptomen.

7. Molekül für seine Anwendung nach Anspruch 6 bei Kindern.

8. Molekül für seine Anwendung nach Anspruch 4 oder 5 zur Behandlung der Narkolepsie oder der idiopathischen Hypersomnie.

9. Molekül für seine Anwendung nach einem der Ansprüche 6 bis 8 als Arzneimittel in einer Dosierung von 200 bis 300 mg pro Tag.

10. Pharmazeutische Zusammensetzung, umfassend das Molekül nach einem der Ansprüche 1 bis 3 als Wirkstoff und einen oder mehrere pharmazeutisch akzeptable Hilfsstoffe.

11. Pharmazeutische Zusammensetzung nach Anspruch 10, angepasst an eine orale Verabreichung.

12. Pharmazeutische Zusammensetzung nach Anspruch 11 in Tablettenform.

13. Pharmazeutische Zusammensetzung, umfassend das Molekül der Formel (I) nach einem der Ansprüche 1 bis 3 und Eisen oder einen oder mehrere Psychostimulanzien oder ein oder mehrere serotoninergische Antidepressiva oder Natriumgammahydroxyburyrat als Wirkstoff.

14. Pharmazeutisches Produkt, umfassend das Molekül der Formel (I) nach einem der Ansprüche 1 bis 3 und Eisen oder einen oder mehrere Psychostimulanzien oder ein oder mehrere serotoninergische Antidepressiva oder Natriumgammahydroxyburyrat als Wirkstoffe als Kombinationsprodukt für eine gleichzeitige, separate oder sequentielle Verabreichung.

15. Verfahren zur Herstellung des Moleküls nach einem der Ansprüche 1 bis 3, umfassend die folgenden Schritte:
(i) Herstellen des Grignard-Reagenz 2
(ii) Herstellen von Fluorbenzhydrol 4 durch Kondensieren des Grignard-Reagenz 2 mit dem Fluorbenzaldehyd 3 oder dem Ethylformiat 10
(iii) Herstellen von Imidat 5 durch Hinzufügen von Thioharnstoff zu dem Fluorbenzhydrol 4
(iv) Verseifung des Imidats 5 und Bildung von Natriumthiolat 6
(v) Hinzufügen des Chloracetamids 7 und Isolieren der Verbindung 8
(vi) Oxidieren des Schwefels der Verbindung 8 in Sulfoxyd.

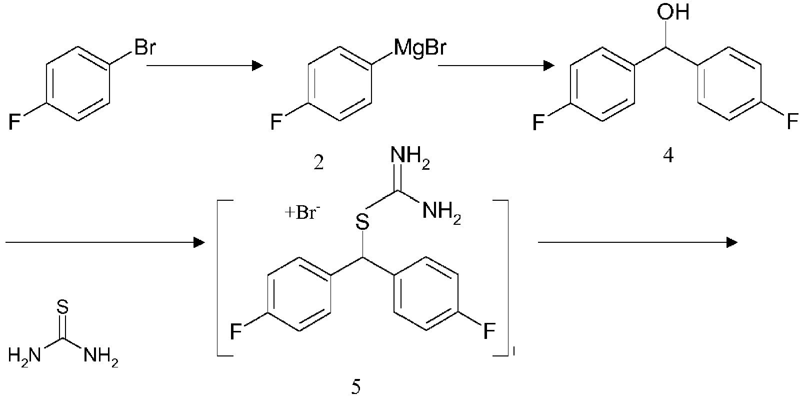

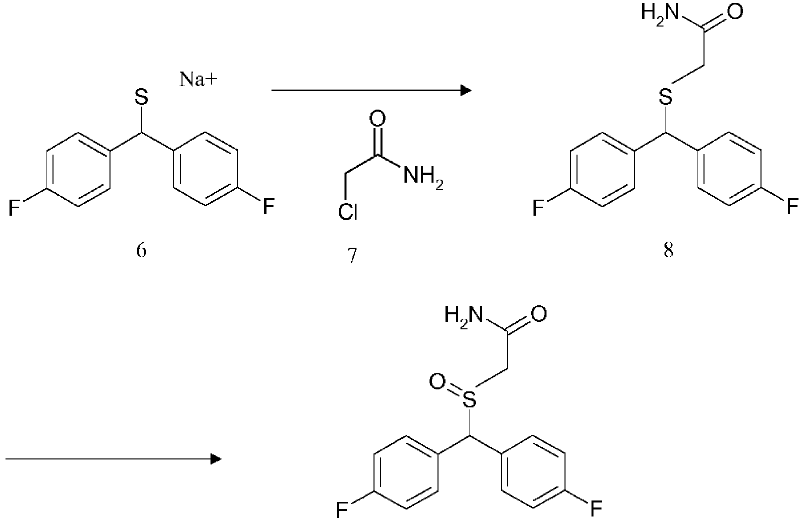

## Claims

1. A molecule of the following formula (I): 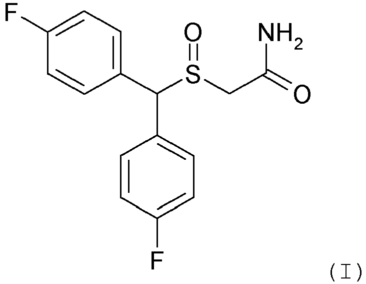 in its optically pure (R) enantiomer form in an enantiomeric excess of more than 95%.

2. The molecule according to claim 1 in its optically pure (R) enantiomer form in an enantiomeric excess of more than 97%.

3. The molecule according to claim 1 in its optically pure (R) enantiomer form in an enantiomeric excess of more than 99%.

4. The molecule according to any one of claims 1 to 3 for use as a drug.

5. The molecule according to claim 4 for use as a drug according to a dosing schedule of 1 to 400 mg per day.

6. The molecule according to claim 4 or 5 for use in the treatment of Attention Deficit/Hyperactivity Disorder (ADHD) and/or associated or comorbid symptoms.

7. The molecule according to claim 6 for use in children.

8. The molecule according to claim 4 or 5 for use in the treatment of narcolepsy or idiopathic hypersomnia.

9. The molecule according to any one of claims 6 to 8 for use as a drug according to a dosing schedule of 200 to 300 mg per day.

10. A pharmaceutical composition comprising the molecule according to any one of claims 1 to 3 as an active ingredient and one or more pharmaceutically acceptable excipients.

11. The pharmaceutical composition according to claim 10, suitable for oral administration.

12. The pharmaceutical composition according to claim 11, in tablet form.

13. The pharmaceutical composition comprising the molecule of formula (I) according to any one of claims 1 to 3 and iron or one or more psychostimulants or one or more serotoninergic antidepressants or sodium gamma-hydroxybutyrate as an active ingredient.

14. A pharmaceutical product comprising the molecule of formula (I) according to any one of claims 1 to 3 and iron or one or more psychostimulants or one or more serotoninergic antidepressants or sodium gamma-hydroxybutyrate as active ingredients as a combination product for simultaneous, separate or sequential administration.

15. A method for preparing the molecule according to any one of claims 1 to 3, including the following steps:
(i) preparation of Grignard reagent 2
(ii) preparation of fluorobenzhydrol 4 by condensation of Grignard reagent 2 with fluorobenzaldehyde 3 or ethyl formate 10
(iii) preparation of imidate 5 by adding thiourea to fluorobenzhydrol 4
(iv) saponification of imidate 5 and formation of sodium thiolate 6
(v) addition of chloroacetamide 7 and isolation of compound 8
(vi) oxidation of the sulfur of compound 8 into sulfoxide.
